# EUROPEAN PATENT SPECIFICATION

(11) **EP 3 821 949 B1**
(45) Date of publication and mention of the grant of the patent: **17.08.2022**
(21) Application number: 19208780.7
(22) Date of filing: 13.11.2019
(51) Int. Cl.: A61P 35/00, A61K 31/337, A61K 31/56, A61K 31/704, A61K 31/7048

(54) **PHARMACEUTICAL COMPOSITION FOR USE IN THE TREATMENT OF A CANCER ASSOCIATED WITH THE ACTIVATION OF GALECTIN-1**
VERFAHREN UND ZUSAMMENSETZUNG ZUR VERWENDUNG IN DER BEHANDLUNG VON KREBS IN ZUSAMMENHANG MIT DER AKTIVIERUNG VON GALECTIN-1
COMPOSITION PHARMACEUTIQUE POUR UNE UTILISATION DANS LE TRAITEMENT D'UN CANCER ASSOCIÉ À L'ACTIVATION DE GALECTINE-1

(43) Date of publication of application: 19.05.2021
(73) Proprietor: Trineo Biotechnology Co. Ltd, New Taipei City 221 (TW)
(72) Inventor: CHEN, Teng-Hai, 733 Tainan City (TW); CHEN, Mon-Tarng, 712 Tainan City (TW); WANG, Chien-Yuan, 718 Tainan City (TW); HUANG, Cheng-Po, 221 New Taipei City (TW); CHEN, Su-Yu, 221 New Taipei City (TW); LIN, Yi-Hsiu, 221 New Taipei City (TW); WANG, Ssu-Chia, 221 New Taipei City (TW); LIAO, Chih-Yuan, 221 New Taipei City (TW)
(74) Representative: Lang, Christian

(56) References cited:
- WO-A1-2006/128378
- US-A1- 2014 357 604
- DARIJA CÖR ET AL: "Antitumour, Antimicrobial, Antioxidant and Antiacetylcholinesterase Effect of Ganoderma Lucidum Terpenoids and Polysaccharides: A Review", MOLECULES, vol. 23, no. 3, 13 March 2018 (2018-03-13) , page 649, XP055692984, DOI: 10.3390/molecules23030649
- KOHNO TOSHITAKA ET AL: "Tubulin polymerization-stimulating activity ofGanodermatriterpenoids", NATURAL MEDICINES - SHOYAKUGAKU ZASSHI, JAPANESE SOCIETY OF PHARMACOGNOSY, TOKYO, JP, vol. 71, no. 2, 11 January 2017 (2017-01-11), pages 457-462, XP036168798, ISSN: 1340-3443, DOI: 10.1007/S11418-017-1072-Y [retrieved on 2017-01-11]
- NA FENG ET AL: "Preparative isolation of ganoderic acid S, ganoderic acid T and ganoderol B from Ganoderma lucidum mycelia by high-speed counter-current chromatography", BIOMEDICAL CHROMATOGRAPHY., vol. 32, no. 10, 31 May 2018 (2018-05-31), page e4283, XP055692966, GB ISSN: 0269-3879, DOI: 10.1002/bmc.4283
- JONATHAN COUSIN ET AL: "The Role of Galectin-1 in Cancer Progression, and Synthetic Multivalent Systems for the Study of Galectin-1", INTERNATIONAL JOURNAL OF MOLECULAR SCIENCES, vol. 17, no. 9, 16 September 2016 (2016-09-16), page 1566, XP055667702, DOI: 10.3390/ijms17091566

## Description

### BACKGROUND OF THE INVENTION

### 1. FIELD OF THE INVENTION

The present disclosure relates to a pharmaceutical composition for use in the treatment or prevention of a cancer associated with activation of galectin-1, in particular myeloma cells, the pharmaceutical composition comprising a novel combination of triterpenoids, ganoderic acid S and ganoderic acid T.

### 2. DESCRIPTION OF RELATED ART

Galectin-1 has been implicated in cancer progression, invasion and metastasis. Evidence indicates that galectin-1 participates in tumor progression by evoking T cell anergy and contributes to cancer-immune escape. Galectin-1 also relates to tumor endothelial cell adhesion and migration; therefore, Galectin-1 may act as a target for therapeutic intervention against cancer. Document US 2014/357604 A1 already teaches a composition suitable for treating breast, lung and colon cancer, which comprises GAS, GAT and other ganoderic acids.

Inventors of the present disclosure unexpectedly identified that certain ganoderic acids in combination with known anti-cancer drugs, paclitaxel or etoposide, could effectively suppress the progression and metastasis of myeloma cells, thus these ganoderic acids may serve as candidates for the development of a medicament for the treatment of myeloma cells.

### SUMMARY

The present disclosure is based, at least in part, unexpected discovery that certain triterpenoids or ganoderic acids isolated from the fruit bodies or mycelia of *Ganoderma lucidum* may suppress or inhibit the growth of some cancerous cells. Thus, these compounds may serve as candidates for the development of a medicament for the treatment and/or prophylaxis of some cancers.

It is the aspect of this disclosure to provide a pharmaceutical composition according to claim 1, which is for use in the treatment or of myeloma in a subject, when being administered to the subject in an effective amount, wherein the composition consists of ganoderic acid S (GAS), ganoderic acid T (GAT) and an anti-cancer drug which is paclitaxel or etoposide; and a pharmaceutically acceptable excipient. According to the present disclosure, the GAS and the GAT are present in the pharmaceutical composition in a weight ratio of about 1:1.85.

The pharmaceutical composition includes another anti-cancer drug, which can be administered before, together with, or after the administration of the present pharmaceutical composition, so as to synergistically suppress the growth of the cancer.

Examples of said another anti-cancer drug, which do not fall under the scope of the claims, include, but are not limited to, docetaxel, camptothecin (CPT), topotecan (TPT), irinotecan (CPT-11), doxorubicin, daunorubicin, epirubicin, fluorouracil, cis-platin, cyclophosphamide, vinblastine, vincristine, ifosfamide, melphalan, mitomycin, methotrexate, mitoxantrone, teniposide, bleomycin, leucovorin, cytarabine, dactinomycin, streptozocin, combretastatin A4-phosphate, and SU5416. In one example, synergistic suppression of ovarian cancer may be achieved when a pharmaceutical composition of GAS and GAT is administered together with the anti-cancer drug of paclitaxel. In another example, synergistic suppression of ovarian cancer is achieved when GAS and GAT are administered together with the anti-cancer drug of doxorubicin.

In use, the subject may be subjected to another treatment that is any of a surgical operation or radiation treatment before, concurrently with or after administering the pharmaceutical composition of this disclosure to the subject.

The details of one or more embodiments of this disclosure are set forth in the accompanying description below. Other features and advantages of the invention will be apparent from the detail descriptions, and from claims.

### BRIEF DESCRIPTION OF THE DRAWINGS

The present description will be better understood from the following detailed description read in light of the accompanying drawings, where:
Figure 1 illustrates the effects of TN-GL-01 composition of present disclosure on ovarian cancer ES-2 cells in accordance with one example of the present disclosure;
Figure 2 illustrates the effects of TN-GL-01 composition on the expression of galectin-1 via western blot analysis in accordance with one example of the present disclosure;
Figure 3 illustrates the respective effects of TN-GL-01 composition, cisplatin, and doxorubicin on paclitaxel-induced cell death in accordance with one example of the present disclosure;
Figure 4 illustrates the respective effects of TN-GL-01 composition, cisplatin, and doxorubicin on doxorubicin-induced cell death in accordance with one example of the present disclosure;
Figure 5A are photographs illustrating the results of immunohistochemical analysis and the tumor appearances in accordance with one example of the present disclosure;
Figure 5B is a bar graph depicting the weight of tumors of Figure 5B;
Figure 6A are photographs illustrating the results of immunohistochemical analysis on CD8-positive cells in accordance with one example of the present disclosure;
Figure 6B is a bar graph depicting the percentage of human immune cells in the cancerous tissues in accordance with one example of the present disclosure;
Figure 7A is a bar graph depicting the amount of paclitaxel retained in the cancerous tissues in accordance with one example of the present disclosure; and
Figure 7B is a bar graph depicting the weight of tumors of Figure 7A.

### DETAILED DESCRIPTION OF PREFERRED EMBODIMENTS

The detailed description provided below in connection with the appended drawings is intended as a description of the present disclosure and is not intended to represent the only forms in which the present disclosure may be constructed or utilized.

### 1. Definition

For convenience, certain terms employed in the context of the present disclosure are collected here. Unless defined otherwise, all technical and scientific terms used herein have the same meaning as commonly understood by one of the ordinary skill in the art to which this invention belongs.

The terms "treatment" and "treating" are used herein to include preventative (e.g., prophylactic), curative, or palliative treatment that results in a desired pharmaceutical and/or physiological effect. Preferably, the effect is therapeutic in terms of partially or completely curing or preventing the growth of tumor cells. Also, the term "treating" as used herein refers to application or administration of the pharmaceutical composition of the present disclosure to a subject, who has a medical condition, a symptom of the condition, a disease or disorder secondary to the condition, or a predisposition toward the condition, with the purpose to partially or completely alleviate, ameliorate, relieve, delay onset of, inhibit progression of, reduce severity of, and/or reduce incidence of one or more symptoms or features of a particular disease, disorder, and/or condition. Treatment may be administered to a subject who does not exhibit signs of a disease, disorder, and/or condition and/or to a subject who exhibits only early signs of a disease, disorder, and/or condition for the purpose of decreasing the risk of developing pathology associated with the disease, disorder, and/or condition. As used herein, the symptom, disease, disorder or condition may be solid tumor or metastatic tumor. Treatment is generally "effective" if one or more symptoms or clinical markers are reduced as that term is defined herein.

The term "prophylaxis" as used herein means prevention against a future event. In the context of prophylaxis against tumor or tumor metastasis that may potentially occur as a consequence of a surgical or diagnostic procedure, the prophylactic administration can occur before, contemporaneous with, and/or after the procedure.

The term "an effective amount" as used herein refers to an amount effective, at dosages, and for periods of time necessary, to achieve the therapeutically desired result with respect to the treatment of cancer.

The terms "compounds," "compositions," "agent" and "medicament" are used interchangeably herein to refer to a compound or a composition of which, when administered to a subject such as a human or an animal induces a desired pharmacological and/or physiological effect by local and/or systemic action.

The terms "administered," "administering" and "administration" are used interchangeably herein to refer means either directly administering a compound or a composition of the present invention, or administering a prodrug, derivative or analog which will form an equivalent amount of the active compound within the body.

The term "subject" or "patient" refers to an animal including the human species that is treatable with the compositions of the present invention. The term "subject" or "patient" intended to refer to both the male and female gender unless one gender is specifically indicated. Accordingly, the term "subject" or "patient" includes, but is not limited to, human, non-human primate such as any mammal, dog, cat, horse, sheep, pig, cow, and etc., preferably a human, which may benefit from treatment by the compound of this disclosure. The terms "subject" and "patient" are used interchangeably in the present disclosure.

Notwithstanding that the numerical ranges and parameters setting forth the broad scope of the invention are approximations, the numerical values set forth in the specific examples are reported as precisely as possible. Any numerical value, however, inherently contains certain errors necessarily resulting from the standard deviation found in the respective testing measurements. Also, as used herein, the term "about" generally means within 10%, 5%, 1%, or 0.5% of a given value or range. Alternatively, the term "about" means within an acceptable standard error of the mean when considered by one of ordinary skill in the art. Other than in the operating/working examples, or unless otherwise expressly specified, all of the numerical ranges, amounts, values and percentages such as those for quantities of materials, durations of times, temperatures, operating conditions, ratios of amounts, and the likes thereof disclosed herein should be understood as modified in all instances by the term "about." Accordingly, unless indicated to the contrary, the numerical parameters set forth in the present disclosure and attached claims are approximations that can vary as desired. At the very least, each numerical parameter should at least be construed in light of the number of reported significant digits and by applying ordinary rounding techniques.

### 2. The pharmaceutical composition

The present disclosure is based, at least in part, unexpected discovery that a novel combination of certain ganoderic acids isolated from the fruit bodies or mycelia of *Ganoderma lucidum,* may suppress or inhibit the expression of galectin-1, which in term, may lead to the suppression of a tumor. Accordingly, the novel combination of the identified ganoderic acids, particularly, the combination of ganoderic acid S (GAS) and ganoderic acid T (GAT), is useful in a therapeutic medicament for the treatment or prophylaxis of some cancers being expected to be mediated by galectin-1. More unexpectedly, a synergistic suppression of the growth and/or metastasis of a tumor may be achieved when the combination of GAS and GAT is administered with an anti-cancer drug, such as etoposide, doxorubicin, and paclitaxel.

More specifically, the present invention provides a pharmaceutical composition for the treatment of myeloma. The pharmaceutical composition includes a triterpenoid that consists of ganoderic acid S (GAS) and ganoderic acid T (GAT); an anti-cancer drug; and a pharmaceutically acceptable excipient. The anti-cancer drug is selected from paclitaxel and etoposide.

In general, GAS and GAT may be respectively isolated from *Ganoderma spp* in accordance with any method known in the art, such as from the fruit bodies of *Ganoderma lucidum* in accordance with the method described by Hirotani et al (Phytochemistry (1987), 26(10), 2797-2803). Alternatively, GAS and GAT may be isolated from the cultivating waste of *Ganoderma lucidum,* particularly from the mycelium remained in the cultivating media of *Ganoderma lucidum* after harvest, in accordance with the method described in Taiwan Patent No. 1381844. The isolation in general involves extracting the *Ganoderma spp* with a solvent, preferably an alcoholic solution, at a temperature above room temperature; followed by column chromatography purification, which may be high performance liquid chromatography (HPLC), reverse phase liquid chromatography and etc.; concentrating and drying the isolated product, until a dried powder is produced.

According to the present disclosure, in aspects which are not part of the claimed invention, the GAS and the GAT may berespectively present in the pharmaceutical composition in a ratio from about 1: 1 to 1:10 by weight, such as 1:1, 1:2, 1:3, 1:4, 1:5, 1:6, 1:7, 1:8, 1:9, and 1:10 by weight. However, according to the present invention , it is chosen that the GAS and the GAT exist in the pharmaceutical composition in a ratio of about 1:2 by weight, more specifically in a ratio of about 1:1.85 by weight.

The triterpenoid of the present disclosure, i.e., the combination of GAS and GAT, is present at a level of about 0.1% to 99% by weight, based on the total weight of the pharmaceutical composition, such as about 0.1, 0.2, 0.3, 0.4, 0.5, 0.6, 0.7, 0.8, 0.9, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, or 99% by weight in the pharmaceutical composition. In some examples, the triterpenoid of the present disclosure is present at a level of at least 1% by weight, based on the total weight of the pharmaceutical composition. In certain examples, the triterpenoid of the present disclosure is present at a level of at least 5% by weight, based on the total weight of the pharmaceutical composition. In still other examples, the triterpenoid of the present disclosure is present at a level of at least 10% by weight, based on the total weight of the pharmaceutical composition. In still yet other examples, the triterpenoid is present at a level of at least 25% by weight, based on the total weight of the pharmaceutical composition.

In some examples, not being part of the present invention, the medicament or the pharmaceutical composition of the present disclosure is used as an adjuvant therapy, in addition to the major cancer therapy, which includes, but is not limited to, surgical operation, radiotherapy, or chemotherapy. The present pharmaceutical composition may be used together with a chemotherapeutic agent.

The medicament or said pharmaceutical composition is prepared in accordance with acceptable pharmaceutical procedures, such as described in Remington's Pharmaceutical Sciences, 17th edition, ed. Alfonoso R. Gennaro, Mack Publishing Company, Easton, Pa (1985). Pharmaceutically acceptable excipients are those that are compatible with other ingredients in the formulation and biologically acceptable.

The pharmaceutical composition of the present disclosure may be administered by any suitable route, for example, orally in capsules, suspensions or tablets or by parenteral administration. Parenteral administration can include, for example, systemic administration such as intramuscular, intravenous, subcutaneous, or intraperitoneal injection. Preferably, the pharmaceutical composition of the present disclosure may be formulated for being administered orally (e.g., dietary) to a subject.

For oral administration, the pharmaceutical composition of the present disclosure may be formulated into tablets containing various excipients such as microcrystalline cellulose, sodium citrate, calcium carbonate, dicalcium phosphate, and glycine; along with various disintegrants such as starch, alginic acid and certain silicates; together with granulation binders like polyvinylpyrrolidone, sucrose, gelatin and acacia. Additionally, lubricating agents such as magnesium stearate, sodium lauryl sulfate and talc may be added. Solid composition may also be employed as fillers in gelatin capsules; preferred materials in this connection also include lactose or milk sugar as well as high molecular weight polyethylene glycols. When aqueous suspensions and/or elixirs are desired for oral administration, the active ingredient may be combined with various sweetening or flavoring agents, coloring matter or dyes, and if so desired, emulsifying and/or suspending agents as well, together with diluents such as water, ethanol, propylene glycol, glycerin and a combination thereof.

For parenteral administration, the medicament or pharmaceutical composition of the present disclosure may be formulated into liquid pharmaceutical compositions, which are sterile solutions, or suspensions that can be administered by, for example, intravenous, intramuscular, subcutaneous, or intraperitoneal injection. Suitable diluents or solvent for manufacturing sterile injectable solution or suspension include, but are not limited to, 1,3-butanediol, mannitol, water, Ringer's solution, and isotonic sodium chloride solution. Fatty acids, such as oleic acid and its glyceride derivatives are also useful for preparing injectables, as are natural pharmaceutically acceptable oils, such as olive oil or castor oil. These oil solutions or suspensions may also contain alcohol diluent or carboxymethyl cellulose or similar dispersing agents. Other commonly used surfactants such as Tweens or Spans or other similar emulsifying agents or bioavailability enhancers that are commonly used in manufacturing pharmaceutically acceptable dosage forms can also be used for the purpose of formulation.

It will be appreciated that the dosage for administering the pharmaceutical composition of the present disclosure will vary from patient to patient not only for the particular route of administration, and the ability of the composition to elicit a desired response in the patient, but also factors such as disease state or severity of the condition to be alleviated, age, sex, weight of the patient, the state of being of the patient, and the severity of the pathological condition being treated, concurrent medication or special diets then being followed by the patient, and other factors which those skilled in the art will recognize, with the appropriate dosage ultimately being at the discretion of the attendant physician. Dosage regimens may be adjusted to provide the improved therapeutic response. An effective amount is also one in which any toxic or detrimental effects of the composition are outweighed by the beneficial effects. Preferably, the compositions of the present disclosure are administered at a dosage and for a time such that the number and/or severity of the symptoms are decreased.

### 3. Use of the pharmaceutical composition in a treatment method

The pharmaceutical composition of the present disclosure may be used in treating a cancer associated with the activation of galectin-1 in a subject. According to the claimed invention, it is for use in the treatment of myeloma in a subject by administering to the subject an effective amount of the pharmaceutical composition described above.

Alternatively or optionally, another agent known to improve the treatment of cancer may be additionally administered before, together with and/or after administering the pharmaceutical composition of this invention. Examples of such agent include, but are not limited to, anti-cancer drug, anti-angiogenesis agent, anti-virus agent, analgesic, anti-anemia drug, cytokine, granulocyte colony-stimulating factor (G-CSF), and anti-nausea drug and the like.

According to the present invention, the present pharmaceutical composition is designed to provide an anti-cancer drug in addition to the GAS and the GAT, in which a synergistic effect on the suppression of cancers is achieved via suppressing the expression of galectin-1. According to the present invention, the anti-cancer drug is paclitaxel or etoposide. Other examples of said anti-cancer drugs, which do not fall under the scope of the claims, include, but are not limited to, docetaxel, camptothecin (CPT), topotecan (TPT), irinotecan (CPT-11), Doxorubicin, daunorubicin, epirubicin, fluorouracil, cis-platin, cyclophosphamide, vinblastine, vincristine, ifosfamide, melphalan, mitomycin, methotrexate, mitoxantrone, teniposide, bleomycin, leucovorin, cytarabine, dactinomycin, streptozocin, combretastatin A4-phosphate, SU5416, and the like. Examples of anti-angiogenesis agent, which do not form part of the invention, include, but are not limited to, DS 4152, TNP-470, SU6668, endostatin, 2-methoxyestradiol, angiostatin, thalidomide, tetrathiomolybdate, linomide, IL-12, and the like. Examples of anti-virus agent, which do not form part of the invention, include, but are not limited to, amantadine, rimantadine, and the like. Examples of analgesic, which do not form part of the invention, include, but are not limited to, paracetamol such as para-acetylaminophenol, non-steroidal antiinflammatory drug (NSAID) such as salicylates, and opioid drugs such as morphine and opium. Example of anti-anemia drug, which does not form part of the invention, includes, but is not limited to, erythropoietin.

Examples of cancers treatable by means of the present pharmaceutical composition which do not form part of the invention, include, but are not limited to, glioblastoma, thyroid cancer, gastrointestinal cancer, liver cancer, lung cancer, breast cancer, pancreatic cancer, melanoma, prostate cancer, ovarian cancer, adenocarcinoma, bladder cancer and kidney cancer.

In one non claimed embodiment, the pharmaceutical composition includes paclitaxel and may be used to be administered to a subject suffering from ovarian cancer. In another example, not falling under the scope of the claims, the pharmaceutical composition is administered together with doxorubicin to a subject suffering from ovarian cancer.

In use, the present pharmaceutical composition described above may be administered to the subject in an amount of about 1 to 120 mg/Kg body weight of the subject per day, such as 1, 10, 20, 30, 40, 50, 60, 70, 80, 90, 100, 110 or 120 mg/Kg/day; preferably, in the amount of about 30 to 110 mg/Kg/day, such as 30, 40, 50, 60, 70, 80, 90, 100or 110 mg/Kg/day; more preferably, in the amount of 80 to 100 mg/Kg/day, such as 80, 90, or 100 mg/Kg/day. It will be appreciated that the dosage of the pharmaceutical composition of the present disclosure will vary from patient to patient not only for the particular route of administration, and the ability of the composition to elicit a desired response in the patient, but also factors such as disease state or severity of the condition to be alleviated, age, sex, weight of the patient, the state of being of the patient, and the severity of the pathological condition being treated, concurrent medication or special diets then being followed by the patient, and other factors which those skilled in the art will recognize, with the appropriate dosage ultimately being at the discretion of the attendant physician. Dosage regimens may be adjusted to provide the improved therapeutic response. An effective amount is also one in which any toxic or detrimental effects of the composition are outweighed by the therapeutically beneficial effects. The dose can be administered in a single dosage, or alternatively in more than one dosage, such as 2, 3, or 4 dosages a day.

For example, the subject may be subjected to a radiation treatment after the administration of the pharmaceutical composition of the present invention.

The present invention will now be described more specifically with reference to the following illustrative examples, which are provided for the purpose of demonstration rather than limitation.

### EXAMPLES

### Materials and Methods

### Cell Culture

Ovarian cancer cell ES-2 and myeloma cell line ARH-77 were used in the present disclosure. Cells was cultured and maintained in Dulbecco's modified Eagle media (DMEM) supplemented with 10 % fetal bovine serum (FBS), 100 IU/ml penicillin, 100 ng/ml streptomycin, 2 mM glutamine, nonessential amino acids and sodium pyruvate in 5% CO₂ at 37 °C. Cells were grown and maintained in Petri dishes (each was 10 cm in diameter) until they reached 80% confluence, and then were subject to cell passages. Briefly, cells were first washed with phosphate buffer solution (PBS, 3 ml) once, then treated with 0.05% Trypsin/0.025% EDTA solution (1 mL) for 5 min so that the attached cells become suspended. The suspended cells were harvested and 2 mL fresh culture media were added therein to neutralize any remaining activity of trypsin. Cell density was adjusted by adding appropriate amounts of culture medium to the cell suspension, which was then used to seed the culture plates. The plates were then returned to the incubator, and cultured in accordance with the steps described above.

### Cell Activity Analysis

Cells were seeded in 96-well plates with a density of 3,000 cells/well and cultured in accordance with the procedures described above. On the day when cell activity analysis was to be conducted, cells were first treated with various concentrations of the triterpenoids of the present disclosure, imatinib or gefitinib for at least 48 hrs, before subjecting them to MTT assay.

### MTT assay

50 µL of the MTT stock solution (5 mg MTT dissolved in 1 ml of sterile PBS) was added to each well, and 50 µL of the MTT stock solution was added to 500 µL of medium alone and used as a negative control. Each sample was incubated at 37°C for 4 hours. Aliquots (450 µl) from each sample were taken to a new well of 48-well culture plate, adding 100µL DMSO, mixing thoroughly using the pipette and reacted at 37°C for 20 min and absorbance was measured at 570 nm.

### Immunohistochemical analysis

Tissues were fixed by 4% paraformaldehyde, embedded in paraffin. Tissue sections of ES2 tumor were processed for antigen retrieval. The samples were incubated using Ku80 (C48E7) rabbit monoclonal antibody, following by incubation with the anti-rabbit HRP secondary antibody. Samples were stained using DAB (brown) and counterstained with hematoxylin (blue).

### Western blot analysis

Samples were respectively subjected to SDS-PAGE, transferred to PVDF membranes. These blots were blocked with 5% nonfat milk powder in TBS-0.1% Tween-20 for 30 minutes, followed by incubation with primary antibodies at 4°C overnight and then horseradish peroxidaseconjugated secondary antibodies (Amersham Biosciences, Piscataway). Imaging of bands was performed using Pierce ECL Western Blotting Substrate (Thermo Fisher Scientific) and ImageQuant LAS-4000 (GE Healthcare).

### Animal Model of Primary Ovarium Tumor

Male NOD/SCID mice about 6 weeks old were used in this experiment. Animals were kept at specific pathogen-free conditions under 12:12 light-dark cycle with food (laboratory rodent diet 5001 purchased from PMI Nutrition International Inc. MO, USA) and water (i.e., distilled water) provided ad libitum, ambient temperature and relative humidity were respectively set at 22±3°C and 50+20%. All procedures involving animal studies of the present disclosure comply with the "Guideline for the Care and Use of Laboratory Animals" issued by The Chinese-Taipei Society of Laboratory Animal Sciences.

The day before tumor inoculation, mice were randomly divided into groups while making sure that the average weight in each group did not differ significantly. The fur on the right hind leg of each animal was then shaved. To inoculate tumor in mice, ovarium cancer cells ES-2 (1× 10⁷ cancer cells/0.1 mL) were injected subcutaneously to NOD/SCID mice, to generate subcutaneous (s.c.) xenograft tumors.

### Example 1 Preparation and characterization of the present pharmaceutical composition

### 1.1 Preparation of the present pharmaceutical composition

A pharmaceutical composition, herein "TN-GL-01 composition" was prepared by mixing the dried powders of GAT (26 mg), GAS (14 mg) and crystallized methylcellulose (MC) (60 mg) in a blender until a homogeneous mixture was obtained.

### 1.2 TN-GL-01 composition inhibited the activity of orarian cancer cells

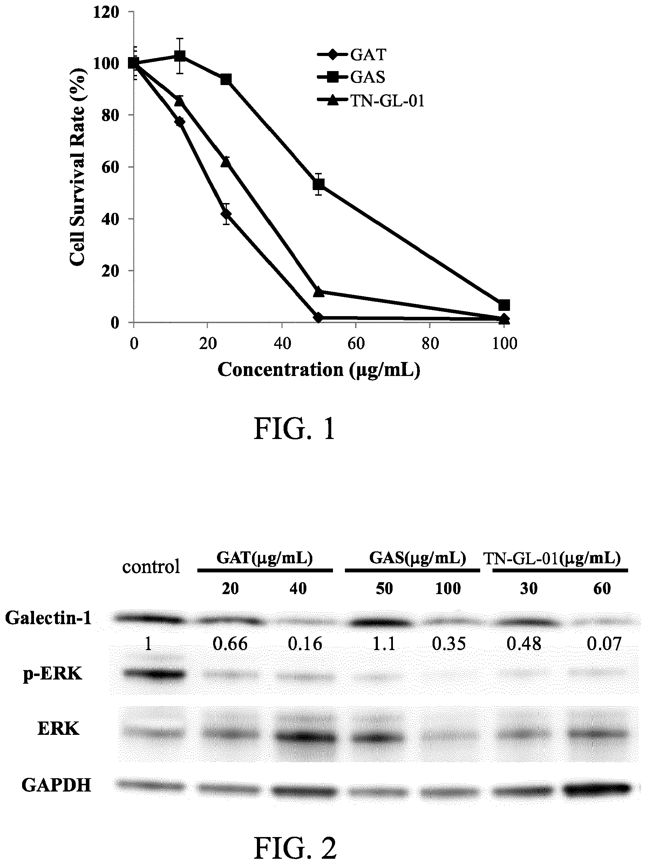

The effects of the TN-GL-01 composition of example 1.1 on ovarian cancer cells (i.e., ES-2 cells) were evaluated by MTT assay in accordance with procedures described in the "Material and Methods" section. Results are provided in Figure 1.

As depicted, 50% inhibition of ES-2 cells by the TN-GL-01 composition, GAS and GAT (IC₅₀) were independently 30.1, 58.3 and 24.5 µg/mL. Thus, as expected, TN-GL-01 composition of example 1.1, GAS, and GAT all exhibited cytotoxic effect on ES-2 cells.

### 1.3 TN-GL-01 composition inhibited galectin-1 expression of ES-2 Cells

In this example, the effects of TN-GL-01 composition on the expression of galectin-1 in ERK (extracellular-signal-regulated kinase) pathway was invesigated via western blot analysis. Results are shown in Figure 2. As depicted, GAS, GAT, and TN-GL-01 composition were all effective in suppressing the expression of galectin-1. Thus, it was concluded that the present TN-GL-01 composition exerted its function through suppression of galectin -1.

### Example 2 In vitro suppression of cancer cells by the combined treatment of TN-GL-01 composition and an anti-cancer drug

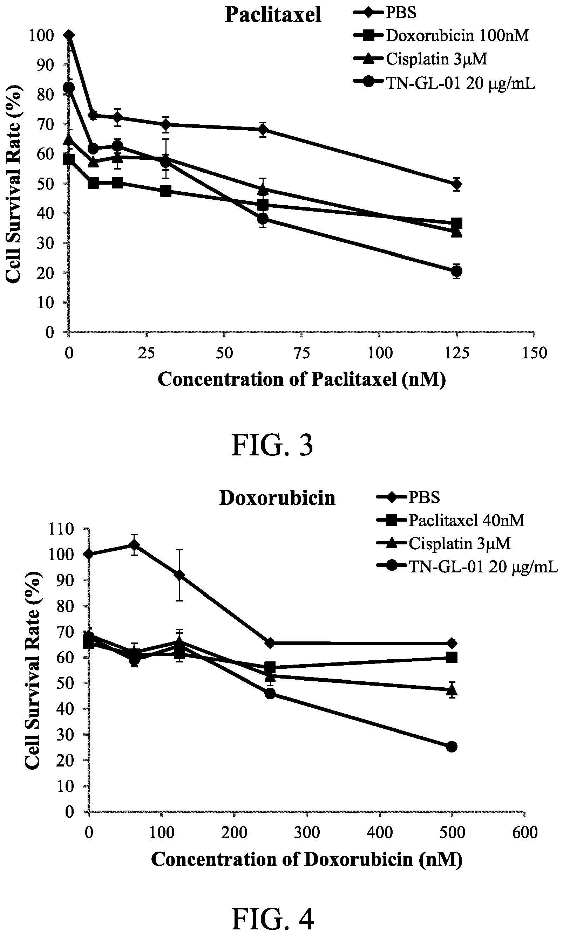

In this example, the effect of TN-GL-01 composition of example 1.1 on the cell killing effect of a chemotherapeutic drug *(i.e.,* etoposide, doxorubicin, cisplatin, and paclitaxel) on myeloma ARH-77 cells was investigated, in which cyclophosphamide served as a comparative drug. Results are summarized in Tables 1 to 3, and Figures 3 and 4.

Reference is first made to Table 1, in which the data indicated that TN-GL-01 composition was more effective than cyclophosphamide in enhancing the etoposide induced cell death in myeoloma ARH-77 cells (IC₅₀ 156 nM vs 238 nM, compared to the control IC₅₀ of 415 nM). Similar results are also observed for another chemotherapeutic drug - doxorubicin (IC₅₀ 50 nM vs 73 nM, compared to the control IC₅₀ of 99 nM)

**Table 1 IC₅₀ of myeoloma ARH-77 cells treated with TN-GL-01 composition and a chemotherapeutic agent**

| Treatment | IC₅₀ (nM) |
|---|---|
| Etoposide | 415 |
| Etoposide + Cyclophosphamide (3mM) | 238 |
| Etoposide + TN-GL-01 (15µg/mL) | 156 |
| Doxorubicin | 99 |
| Doxorubicin + Cyclophosphamide (3mM) | 73 |
| Doxorubicin + TN-GL-01 (15µg/mL) | 50 |

To determine the effects of combined use of TN-GL-01 and any other three drugs including paclitaxel, doxorubicin, and cisplatin, the statistical differences between expected value and oberved value of the survival rate were calculated. If no significantance existed between the expected and observed values, the effect is termed"additivity (p > 0.05)". If the observed value of the survival rate was significantly lower than the expected value, the given effect in combination is termed "synergy (p < 0.05)". Additionally, antagonism indicates that a statistically higher observed value of survival rate was found when comparing to its expected value of survival rate. The expected value was determined by multifying the survival rate of each drug in specific dose. The obtained value and value of each survival rate were measured by MMT assay (Fig. 3 and Fig. 4). The results were prsented as mean ± standard deviation (SD) as shown in Tables 2 and 3.

Referring to Figure 3, which depicts the respective effects of TN-GL-01 composition, cisplatin, and doxorubicin on paclitaxel induced cell death. Among the three anti-cancer agents (i.e.,TN-GL-01 composition, cisplatin, and doxorubicin), TN-GL-01 composition was most effective in enhancing the paclitaxel induced cell death (Figure 3). Surprisingly, synergistic supprission of the ARH-77 cells was observed when cells were treated with the combinaiton of TN-GL-01 composition (20µg/mL) and paclitaxel (62.5 nM and 125 nM, respectively) (see Table 2).

Similarly, TN-GL-01 composition was also most effective in enhancing the doxorubicin-induced cell death, as compared to that of cisplatin or paclitaxel (Figure 4). Also, synergistic supprission of the ARH-77 cells was observed when cells were treated with the combination of TN-GL-01 composition (20µg/mL) and doxorubicin at the concentration up to 500 nM (Table 3), whereas in the case when the concentration of doxorubicin was no more than 200 nM, then only additive or antagonic effect was observed (Table 3).

**Table 2**

| Combination | **Cell Survival Rate** | | |
|---|---|---|---|
| | Expected value | observed value | result |
| Paclitaxel (62.5 nM)+ TN-GL-01 (20 µg/mL) | 0.68 × 0.82 = 0.56±0.03 | 0.38±0.02 (p=0.002) | **Synergy** |
| Paclitaxel (125 nM)+ TN-GL-01 (20 µg/mL) | 0.50 × 0.82 = 0.41±0.03 | 0.20±0.03 (p=0.0008) | **Synergy** |
| | | | |
| Paclitaxel (62.5 nM)+ Doxorubicin (100 nM) | 0.68 × 0.58 = 0.39±0.02 | 0.43±0.03 (p=0.18) | Additivity |
| Paclitaxel (125 nM)+ Doxorubicin (100 nM) | 0.50 × 0.58 = 0.29±0.01 | 0.38±0.01 (p=0.003) | Antagonism |
| | | | |
| Paclitaxel (62.5 nM)+ Cisplatin (3 µM) | 0.68 × 0.65 = 0.44±0.04 | 0.48±0.04 (p=0.27) | Additivity |
| Paclitaxel (125 nM)+ Cisplatin (3 µM) | 0.50 × 0.65 = 0.33±0.03 | 0.34±0.01 (p=0.45) | Additivity |

**Table 3**

| combination | **Cell Survival Rate** | | |
|---|---|---|---|
| | Expected value | Observed value | result |
| Doxorubicin (250 nM)+TN-GL-01 (20 µg/mL) | 0.66 × 0.68 = 0.44±0.02 | 0.46±0.02 (p=0.46) | Additivity |
| Doxorubicin (500 nM)+TN-GL-01 (20 µg/mL) | 0.66 × 0.68 = 0.44±0.03 | 0.25±0.01 (p=0.0004) | **Synergy** |
| | | | |
| Doxorubicin (250 nM)+ Paclitaxel (40 µM) | 0.66 × 0.65 = 0.43±0.02 | 0.56±0.01 (p=0.0004) | Antagonism |
| Doxorubicin (500 nM)+ Paclitaxel (40 µM) | 0.66 × 0.65 = 0.43±0.02 | 0.60±0.01 (p=0.0003) | Antagonism |
| | | | |
| Doxorubicin (250 nM)+ Cisplatin (3 µM) | 0.66 × 0.69 = 0.45±0.01 | 0.53±0.04 (p=0.03) | Antagonism |
| Doxorubicin (500 nM)+ Cisplatin (3 µM) | 0.66 × 0.69 = 0.45±0.02 | 0.47±0.03 (p=0.31) | Additivity |

Taken together, the results from this example suggested that in addition to its own cytotoxicity on cancerous cells, the present TN-GL-01 composition may result in synergistic suppression of cancerous cells when administered together with another chemotherapeutic agent, such as paclitaxel and doxorubicin.

### EXAMPLE 3 In vivo suppression of ovarian tumor by the combined treatment of TN-GL-01 composition and paclitaxel (reference example)

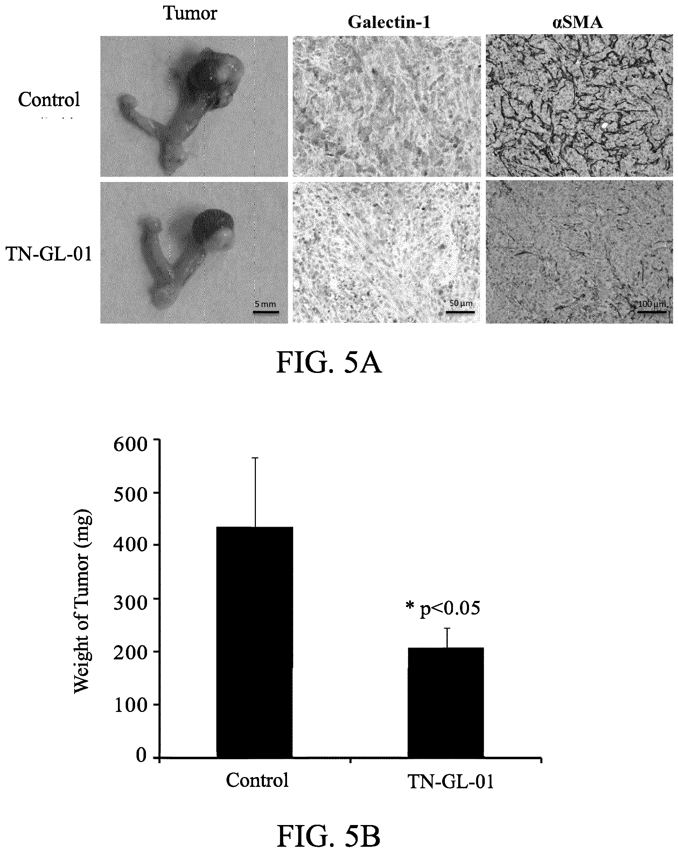

In this example, ovarian tumor was innoculated and inducted in mice in accordance with procedures described in the "Material and Methods"section. Mice were randomly assiged into different groups and treated with the TN-GL-01 composition of example 1.1 (300 mg/Kg), paclitaxel (10 mg/Kg) or a combinaiton of paclitaxel (10 mg/Kg or 20 mg/Kg) and the TN-GL-01 composition (300 mg/Kg) via intraperitoneal injection. Mice were then sacrified, the expression of galectin-1 in stromal cells, the amount of paclitaxel retained in the cancerous tissues, as well as the level of CD-8 positive cells in the test animals were measured. Results are shown in Figures 5 to 7.

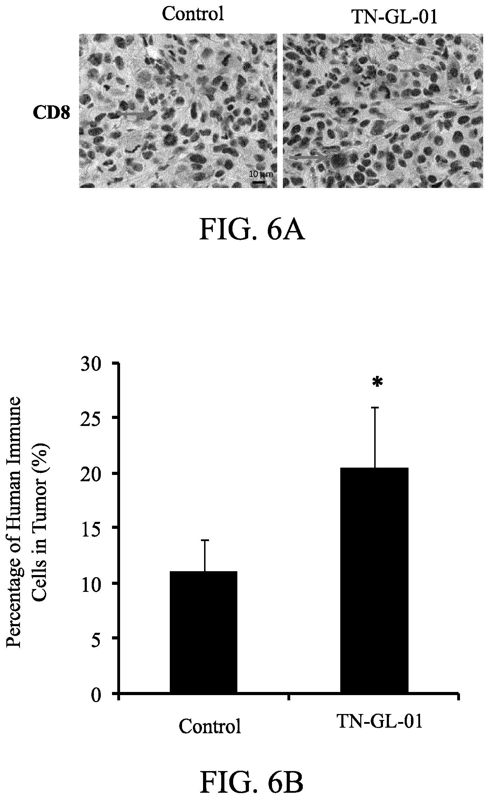

By appearance, the growth of ovarian tumor excised from the test animals treated with theTN-GL-01 composition of example 1.1 was significantly suppressed, as the weight of tumor was 52% less than that of the control animals (Figures 5A and 5B). Further, treatment with TN-GL-01 composition reduced the levels of galectin-1 in storma cells, which was consistent with the finding in example 1.3. As to the expression of α-SMA, the number of stromal cells positively expressed α-SMA was lower than that of the control (Figure 5A), which suggested that the TN-GL-01 composition might have changed the tumor microenvironment that facilitated the infiltration of immunce cells, and was consistent with the finding depicted in Figures 6A and 6B, in which an increased number of immune cells (i.e., CD-8) was found in TN-GL-01 compositoin treated ovarian tumor.

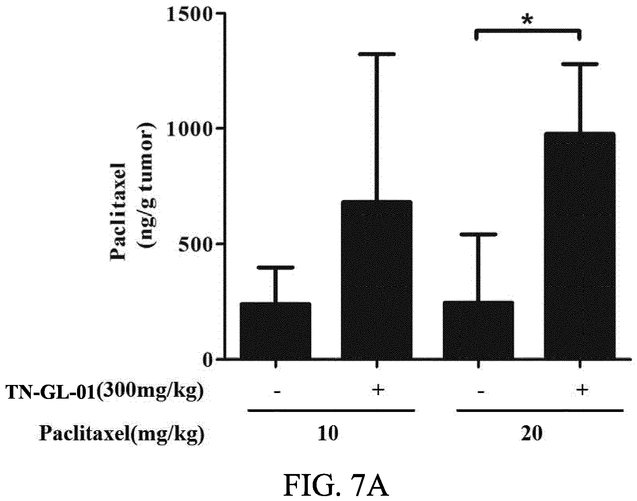

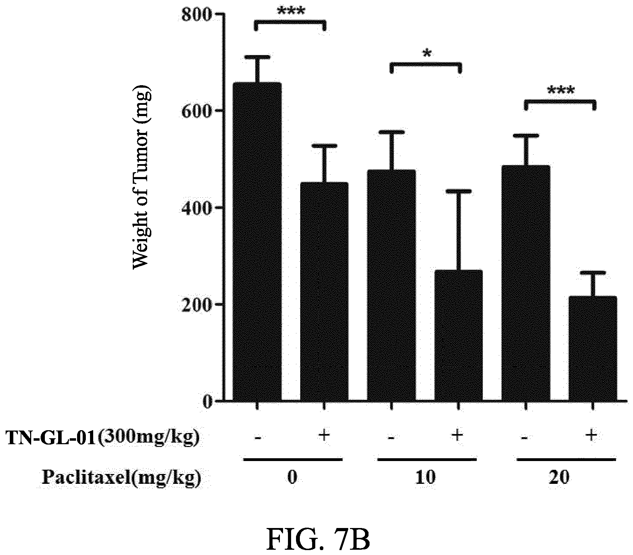

It was also found that TN-GL-01 composition enhanced the amount of a chemotherapeutic agent (i.e, paclitaxel) retained in the tumor tissue. As depicted in Figure 7A, the amount of paclitaxel remained in the tumor tissue was significantly higher in tumors treated with TN-GL-01 (300 mg/Kg) and paclitaxel (10 or 20 mg/Kg). Accordingly, the tumor weight was also significantly reduced in tumors treated with the combination of TN-GL-01 (300 mg/Kg) and paclitaxel (10 or 20 mg/Kg) (Figure 7B).

Taken together, results of the present disclosure indicate that TN-GL-01 composition is suitable for the development of a medicament or an adjuvant for treating cancers.

It will be understood that the above description is given by way of example only and that various modifications may be made by those with ordinary skill in the art. The above specification, examples and data provide a complete description of the structure and use of exemplary embodiments of the invention. Although various embodiments of the invention have been described above with a certain degree of particularity, or with reference to one or more individual embodiments, those with ordinary skill in the art could make numerous alterations to the disclosed embodiments without departing from the scope of the present disclosure.

## Claims

1. A pharmaceutical composition for use in the treatment of myeloma in a subject, when administered to the subject in an effective amount, wherein the pharmaceutical composition comprises ganoderic acid S (GAS) and ganoderic acid T (GAT) in a weight ratio of 1:1.85; an anti-cancer drug being paclitaxel or etoposide and a pharmaceutically acceptable excipient.

2. The pharmaceutical composition for use according to claim 1, wherein the anti-cancer drug is paclitaxel.

3. The pharmaceutical composition for use according to claim 1, wherein the anti-cancer drug is etoposide.

## Patentansprüche

1. Pharmazeutische Zusammensetzung zur Verwendung in der Behandlung von Myelomen in einem Subjekt bei Verabreichung in einer wirksamen Menge an das Subjekt, wobei die pharmazeutische Zusammensetzung Ganodersäure S (GAS) und Ganodersäure T (GAT) in einem Gewichtsverhältnis von 1:1,85, ein Antikrebs-Medikament, welches Paclitaxel oder Etoposid ist, und einen pharmazeutisch verträglichen Hilfsstoff umfasst.

2. Pharmazeutische Zusammensetzung zur Verwendung nach Anspruch 1, bei welcher das Antikrebs-Medikament Paclitaxel ist.

3. Pharmazeutische Zusammensetzung zur Verwendung nach Anspruch 1, bei welcher das Antikrebs-Medikament Etoposid ist.

## Revendications

1. Composition pharmaceutique pour utilisation dans le traitement d'un myélome chez un sujet, lorsqu'elle est administrée au sujet en une quantité efficace, dans laquelle la composition pharmaceutique comprend de l'acide ganodérique S (GAS) et de l'acide ganodérique T (GAT) en un rapport pondéral de 1 : 1,85 ; un médicament anticancéreux étant le paclitaxel ou l'étoposide et un excipient pharmaceutiquement acceptable.

2. Composition pharmaceutique pour utilisation selon la revendication 1, dans laquelle le médicament anticancéreux est le paclitaxel.

3. Composition pharmaceutique pour utilisation selon la revendication 1, dans laquelle le médicament anticancéreux est l'étoposide.
